# EUROPÄISCHE PATENTSCHRIFT

(11) **EP 1 001 937 B1**
(45) Veröffentlichungstag und Bekanntmachung des Hinweises auf die Patenterteilung: **11.06.2003**
(21) Anmeldenummer: 98941368.7
(22) Anmeldetag: 20.07.1998
(51) Int. Cl.: C07D 231/08, C07B 39/00

(54) **VERFAHREN ZUR HERSTELLUNG VON 2-(3-PYRAZOLYL-OXYMETHYLEN)NITROBENZOLEN**
METHOD FOR PRODUCING 2-(3-PYRAZOLYL-OXYMETHYLENE) NITROBENZENES
PROCEDE POUR LA PREPARATION DE 2-(3-PYRAZOLYL- OXYMETHYLENE)-NITROBENZENE

(30) Priorität: 30.07.1997 DE 19732692
(43) Veröffentlichungstag der Anmeldung: 24.05.2000
(73) Patentinhaber: BASF AKTIENGESELLSCHAFT, 67056 Ludwigshafen (DE)
(72) Erfinder: WINGERT, Horst, D-68159 Mannheim (DE); GÖTZ, Norbert, D-67547 Worms (DE); KEIL, Michael, D-67251 Freinsheim (DE); KLINTZ, Ralf, D-67269 Grünstadt (DE); VOGELBACHER, Uwe, Josef, D-67071 Ludwigshafen (DE); WAHL, Josef, D-67105 Schifferstadt (DE)
(74) Vertreter: Münch, Volker, Dr.
(86) Internationale Anmeldenummer: EP9804490
(87) Internationale Veröffentlichungsnummer: WO99006373

(56) Entgegenhaltungen:
- WO-A-96/01256
- DE-A- 2 614 485
- E.M.M. VAN DEN BERG ET AL.: RECUEIL DES TRAVAUX CHIMIQUES DES PAYS-BAS., Bd. 107, Nr. 2, 1988, Seiten 73-81, XP002087429 AMSTERDAM NL

## Beschreibung

Die vorliegende Erfindung betrifft ein Verfahren zur Herstellung von 2-(3-Pyrazolyl-oxymethylen)-nitrobenzolderivaten der Formel I 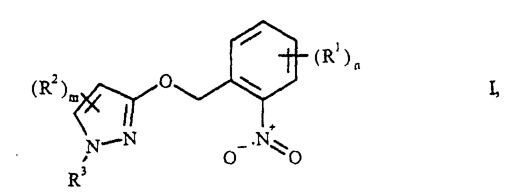 in der die Substituenten und Indices die folgende Bedeutung haben:
- R¹: Halogen;
ggf. subst. Alkyl oder Alkoxy;
- R²: Cyano, Halogen, Alkyl, Halogenalkyl, Alkoxy, Alkylthio oder Alkoxycarbonyl;
- R³: ggf. subst. Alkyl, Alkenyl oder Alkinyl;
ggf. subst. gesättigtes oder ein- oder zweifach ungesättigtes Carbocyclyl oder Heterocyclyl;
ggf. subst. Aryl oder Heteroaryl;
- m: 0, 1 oder 2, wobei die Substituenten R² verschieden sein können, wenn m größer als 1 ist;
- n: 0,1,2,3 oder 4, wobei die Substituenten R¹ verschieden sein können, wenn n größer als 1 ist;
durch Bromierung eines o-Nitrotoluols der Formel II 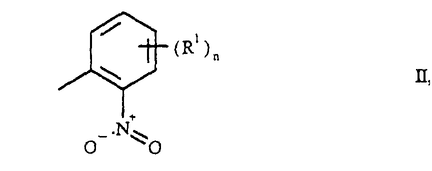 in der R¹ die vorgenannte Bedeutung besitzt, zum o-Nitrobenzylbromid der Formel III 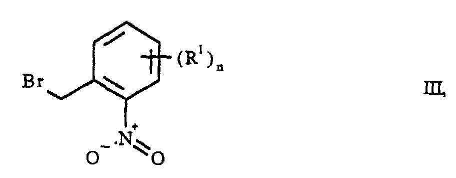 in Gegenwart eines unpolaren, aprotischen Lösungsmittels und anschließender Umsetzung der so erhaltenen Lösung von III mit einem 3-Hydroxypyrazol der Formel IV 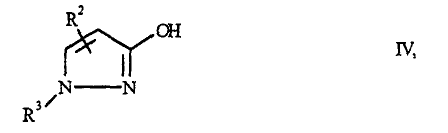 in der R² und R³ die vorgenannte Bedeutung haben, in Gegenwart einer Base.

In der Literatur sind zahlreiche Verfahren zur Herstellung von o-Nitrobenzylbromiden der Formel III ausgehend von o-Nitrotoluolderivaten II beschrieben. Die Seitenkettenbromierung gelingt in vielen Fällen aufgrund der Desaktivierung durch die Nitrogruppe erst bei Temperaturen oberhalb von 100°C und unter Druck. Bedingungen, die in Anbetracht der geringen thermischen Stabilität der o-Nitrobenzylbromide von Nachteil und sicherheitstechnisch problematisch sind (siehe Z. Chem. 12 (1972) 139).

In der WO 96/01256 wird die Herstellung von 2-(3-Pyrazolyl-oxymethylen)-nitrobenzol derivaten I ausgehend von o-Nitrobenzylbromiden III allgemein beschrieben. Auf die technische Herstellung von III wird in dieser Schrift nicht näher eingegangen. Auch gibt die Schrift keine Hilfestellung wie bei einer großtechnischen Realisierung des beschriebenen Verfahrens ein sicherer Umgang mit III bewerkstelligt werden soll. Der Umgang mit technischen Mengen von III ist wegen der tränen- und schleimhautreizenden Wirkung von III und der bereits angesprochenen thermischen Instabilität von III problematisch.

Aufgabe der vorliegenden Erfindung war es daher einen großtechnisch anwendbaren Weg zu 2-(3-Pyrazolyl-oxymethylen)-nitrobenzolderivaten I zu finden, der einerseits die schwierige arbeits- und sicherheitstechnische Problematik beim Umgang mit III löst und andererseits das gewünschte Produkt I in guter Ausbeute und Reinheit liefert. Verbindungen der Formel I sind wichtige Zwischenprodukte zur Herstellung u.a. der in der WO 96/01256 beschriebenen fungiziden Wirkstoffe.

Demgemäß wurde das eingangs erwähnte Verfahren gefunden, das dadurch gekennzeichnet ist, daß die bei der Bromierung anfallende Lösung des o-Nitrobenzylbromids III im verwendeten Lösungsmittel ohne Zwischenisolierung des o-Nitrobenzylbromids III direkt mit IV weiter umgesetzt wird.

Überraschenderweise liefert das erfindungsgemäße Verfahren die gewünschte Verbindung I in guter Ausbeute und in hervorragender Reinheit. Dies war nicht zu erwarten, da die Bromierung stets unter Bildung nicht unerheblicher Mengen von o-Nitrobenzalbromid V abläuft, welches mit 3-Hydroxypyrazolen IV zu bis-O-alkylierten Acetalen der Formel VI abreagieren kann. 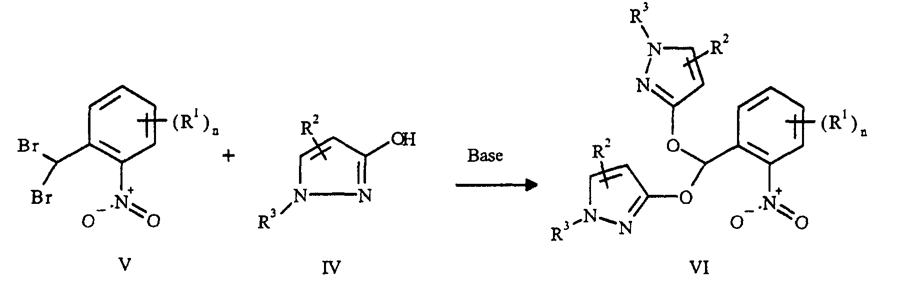 Wird das 3-Hydroxypyrazol IV, als teurere Komponente, in Bezug auf das o-Nitrobenzylbromid III in äquimolaren Mengen oder gar im Unterschuß eingesetzt, so läßt sich die Bildung von VI nahezu vollständig unterdrücken. Die Selektivität der Alkylierungsreaktion ist überraschend, vielmehr wäre man bei den Substraten o-Nitrobenzylbromid III und o-Nitrobenzalbromid V von einer vergleichbaren Reaktivität ausgegangen.

Das erfindungsgemäße Verfahren läßt sich zur Herstellung von 2-(3-Pyrazolyl-oxymethylen)-nitrobenzolderivaten der Formel I anwenden. Die vorstehend für die Substituenten R¹ bis R³ in der Formel I genannten Bedeutungen stellen Sammelbegriffe für individuelle Aufzählungen der einzelnen Gruppenmitglieder dar. Sämtliche Alkylteile können geradkettig oder verzweigt sein. Halogenierte Substituenten tragen vorzugsweise 1 bis 6 gleiche oder verschiedene Halogenatome.

Im einzelnen bedeuten beispielsweise:
Halogen: Fluor, Chlor, Brom und Jod;
Alkyl oder die Alkylteile von Alkoxy, Alkoxycarbonyl und Alkylthio: gesättigte, geradkettige oder verzweigte Kohlenwasserstoffreste, insbesondere mit 1 bis 10 Kohlenstoffatomen, z.B. C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
Alkenyl: ungesättigte, geradkettige oder verzweigte Kohlenwasserstoffreste, insbesondere mit 2 bis 10 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
Alkinyl: geradkettige oder verzweigte Kohlenwasserstoffgruppen, insbesondere mit 2 bis 20 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-2-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl- 1-pentinyl, 3-Methyl-4-pentinyl, 4-Methyl-1-pentinyl, 4-Methyl-2-pentinyl, 1,1-Dimethyl-2-butinyl, 1,1-Di-methyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
Halogenalkyl: geradkettige oder verzweigte Alkylgruppen mit 1 bis 4 Kohlenstoffatomen (wie vorstehend genannt), wobei diese in Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
gesättigtes oder ein- oder zweifach ungesättigtes Carbocyclyl oder Heterocyclyl: beispielsweise Carbocyclen wie Cyclopropyl, Cyclopentyl, Cyclohexyl, Cyclopent-2-enyl, Cyclohex-2-enyl oder Heterocyclyl wie 2-Tetrahydrofuranyl, 2-Tetrahydrothienyl, 2-Pyrrolidinyl, 3-Isoxazolidinyl, 3-Isothiazolidinyl, 1,3,4-Oxazolidin-2-yl, 2,3-Dihydrothien-2-yl, 4,5-Isoxazolin-3-yl, 3-Piperidinyl, 1,3-Dioxan-5-yl, 4-Piperidinyl, 2-Tetrahydropyranyl, 4-Tetrahydropyranyl;
Aryl oder Heteroaryl: beispielsweise Phenyl und Naphthyl, vorzugsweise Phenyl oder 1- oder 2-Naphthyl, und Hetarylreste, beispielsweise 5-Ring Heteroaromaten enthaltend ein bis drei Stickstoffatome und/oder ein Sauerstoff- oder Schwefelatom wie 2-Furyl, 3-Furyl, 2-Thienyl, 3-Thienyl, 1-Pyrrolyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,5-Triazol-3-yl, 1,2,3-Triazol-4-yl, 1,2,3-Triazol-5-yl, 1,2,3-Triazol-4-yl, 5-Tetrazolyl, 1,2,3,4-Thiatriazol-5-yl und 1,2,3,4-Oxatriazol-5-yl, insbesondere 3-Isoxazolyl, 5-Isoxazolyl, 4-Oxazolyl, 4-Thiazolyl, 1,3,4-Oxadiazol-2-yl und 1,3,4-Thiadiazol-2-yl;
Der Zusatz "ggf. subst" in Bezug auf Alkyl-, Alkenyl- und Alkinylgruppen und in Bezug auf Aryl und Hetaryl soll zum Ausdruck bringen, daß diese Gruppen partiell oder vollständig halogeniert sein können (d.h. die Wasserstoffatome dieser Gruppen können teilweise oder vollständig durch gleiche oder verschiedene Halogenatome wie vorstehend genannt (vorzugsweise Fluor, Chlor und Brom, insbesondere Fluor und Chlor) ersetzt sein) und/oder einen bis drei, insbesondere einen, der folgenden Reste tragen können:
   C₁-C₆-Alkoxy, C₁-C₆-Halogenalkoxy, C₁-C₆-Alkylthio, C₁-C₆-Halogenalkylthio, C₁-C₆-Alkylamino, Di-C₁-C₆-Alkylamino, C₂-C₆-Alkenyloxy, C₂-C₆-Halogenalkenyloxy, C₂-C₆-Alkinyloxy, C₂-C₆-Halogenalkinyloxy, C₃-C₆-Cycloalkyl, C₃-C₆-Cycloalkoxy, C₃-C₆-Cycloalkenyl, C₃-C₆-Cycloalkenyloxy, C₁-C₆-Alkylcarbonyl, C₁-C₆-Alkoxycarbonyl, C₁-C₆-Alkylaminocarbonyl, Di-C₁-C₆-Alkylaminocarbonyl, C₁-C₆-Alkylcarbonyloxy, C₁-C₆-Alkylcarbonylamino. Aryl und Hetaryl können zusätzlich zu den bereits genannten einen bis drei der folgenden Reste tragen: C₁-C₆-Alkyl und C₁-C₆-Halogenalkyl.

Geeignete Lösungsmittel für das erfindungsgemäße Verfahren sind solche, die sich bei der Bromierung wie der anschließenden Alkylierung als inert erweisen, wie beispielsweise aromatische Kohlenwasserstoffe wie Benzol, tert.-Butylbenzol, tert.-Amylbenzol oder halogenierte Kohlenwasserstoffe wie Methylenchlorid, 1,2-Dichlorethan, Chloroform, Tetrachlorkohlenstoff, ortho- oder para-Dichlorbenzol, 1,2,4-Trichlorbenzol und insbesondere Chlorbenzol.

Die im erfindungsgemäßen Verfahren eingesetzten ortho-Nitrotoluole II sind zumeist käuflich erwerblich oder nach bekannten Verfahren einfach zugänglich (z.B. Organikum Barth Verlagsgesellschaft (1993) 320ff).

Für die Bromierung der ortho-Nitrotoluole II lassen sich Bromierungsmittel wie elementares Brom, oder Bromsalze wie beispielsweise Natriumbromid u.a. sowie Bromwasserstoff, bevorzugt in Form der Bromwasserstoffsäure einsetzen, wobei die beiden letzteren bevorzugt in Gegenwart eines Oxidationsmittels verwendet werden. Besonders bevorzugt wird eine technische azeotrope Mischung der Bromwasserstoffsäure (ca. 47%ig).

Für die Oxidation des Bromwasserstoffs, bzw. der Bromid-Ionen sind beispielsweise Oxidationsmittel wie Persäuren, Peroxide, Chlorbleichlauge, Chlor, Natriumbromat und Kaliumperoxodisulfat geeignet, besonders geeignet ist Wasserstoffperoxid.

In einer bevorzugten Ausführungsform des erfindungsgemäßen Verfahrens wird mit solchen Mengen an Oxidationsmittel gearbeitet, daß auch der bei der Reaktion gebildete Bromwasserstoff wieder oxidiert wird. Bevorzugt werden pro Bromidäquivalent 1,5 bis 2,0 Äquivalente (Val) des Oxidationsmittels zugesetzt. Verwendet man hingegen elementares Brom als Bromquelle, so kann wahlweise auf die Verwendung eines Oxidationsmittels verzichtet werden, oder aber, falls man den bei der Reaktion gebildeten Bromwasserstoff oxidieren will, so genügt es, 0,5 bis 1,0 Äquivalente (bezogen auf Brom) eines Oxidationsmittels zusetzen. Auf diese Weise läßt sich die Einsatzmenge an Bromierungsmittel nahezu halbieren.

Das Bromierungsmittel wird im allgemeinen in einem Molverhältnis von 0,7 bis 1,3 und bevorzugt in einem Molverhältnis von 0,9 bis 1,0 zum eingesetzten o-Nitrotoluol II eingesetzt.

Als Initiatoren für die Generierung der für die Reaktion erforderlichen Bromradikale werden im erfindungsgemäßen Verfahren bevorzugt Azoverbindungen wie Azocarbonsäureester und Azocarbonsäurenitrile eingesetzt. Besonders bevorzugt wird Azoisobutyronitril verwendet.

Die Initiatoren werden im allgemeinen in einer Konzentration von 0,1 bis 20 mol.-%, bezogen auf das o-Nitrololuol (II), und bevorzugt in einer Konzentration von 1 bis 10 mol.-% dem Reaktionsgemisch zugesetzt.

Die Bromierung wird bei Temperaturen von 20 bis 100°C, bevorzugt bei 20 bis 80°C durchgeführt. Die optimale Reaktionstemperatur richtet sich zum einen nach der thermischen Stabilität des eingesetzten o-Nitrotoluols II und des daraus gewonnenen Produkts III und zum anderen nach der Zerfallstemperatur des Initiators. Die folgende Tabelle gibt eine Übersicht verschiedener Initiatoren, der jeweiliger Struktur und dazugehöriger 10-h-Halbwertszeit-Zerfalltemperatur. Bevorzugt wird bei einer Reaktionstemperatur knapp über oder unterhalb der 10-h-Halbwertszeit-Zerfalltemperatur des Initiators gearbeitet (± 10°C).

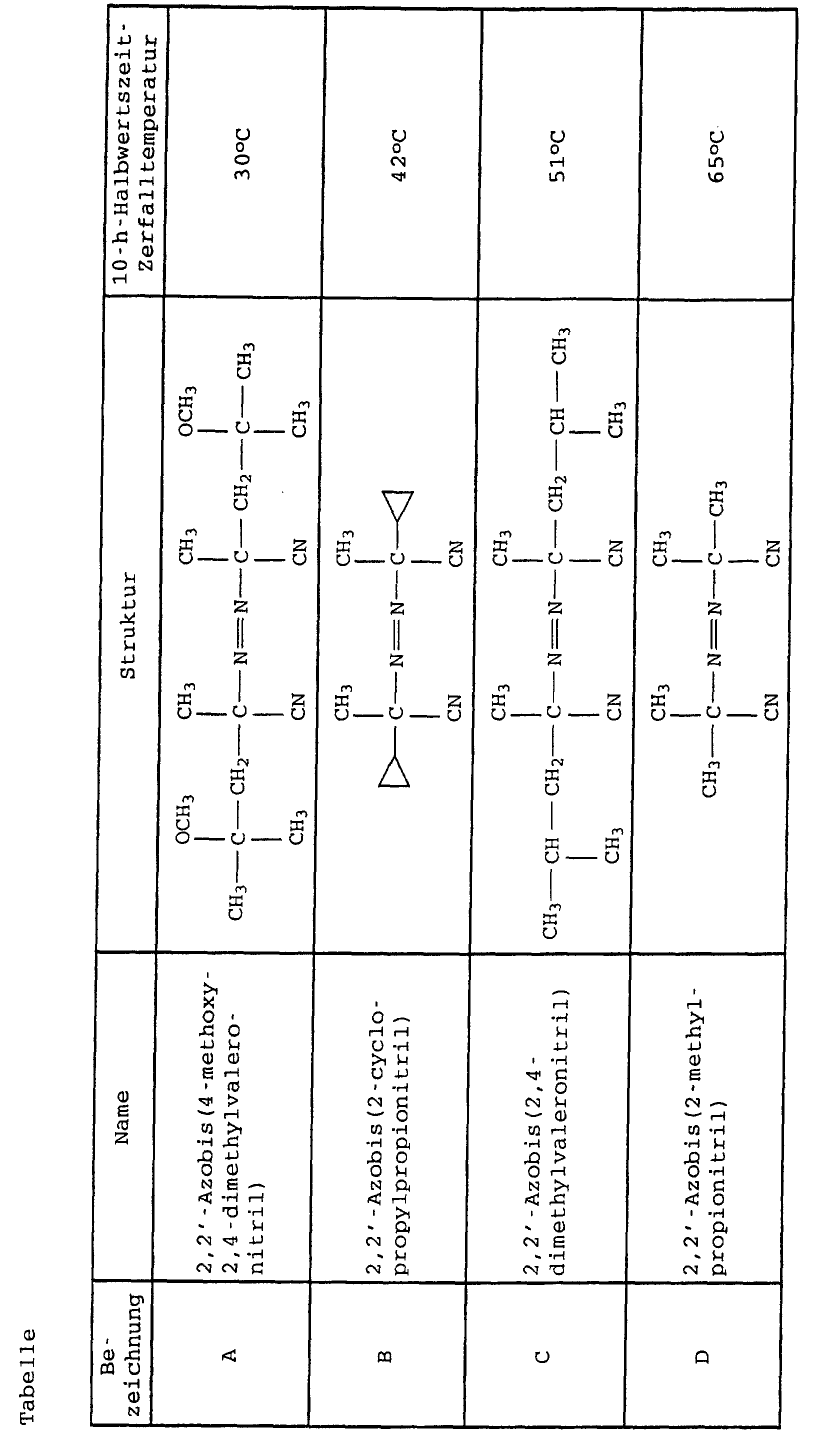

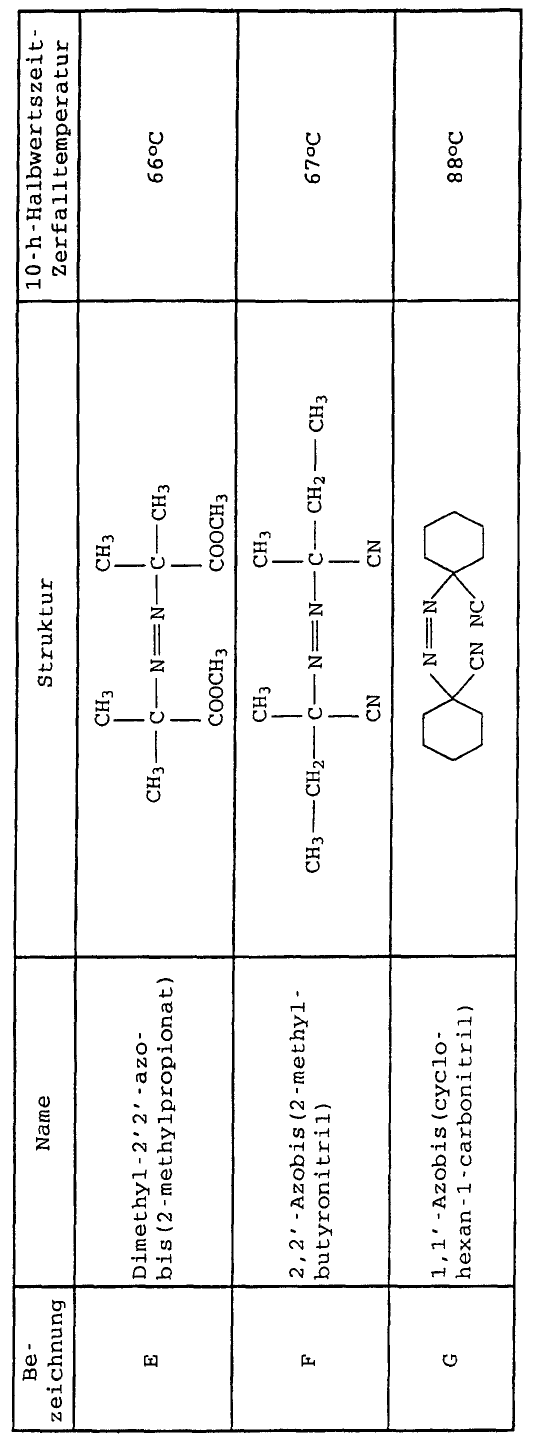

Bevorzugt wird die Bromierung in einem Zweiphasensystem ausgeführt. Im Zweiphasensystem wird im allgemeinen die Lösung des Bromsalzes in Wasser oder bevorzugt die Bromwasserstoffsäure zusammen mit dem verwendeten Lösungsmittel und gegebenenfalls der Initiator bzw. ein Teil der Initiatormenge vorgelegt. Das Reaktionsgemisch wird auf Reaktionstemperatur gebracht und anschließend wird das Nitrotoluol II gegebenenfalls in Gegenwart des Initiators kontinuierlich oder portionsweise im Verlauf von einer halben bis mehreren Stunden zudosiert. Parallel zur Dosierung von II erfolgt die Dosierung des Oxidationsmittel im allgemeinen dergestalt, daß in der Reaktionsmischung kein überschüssiges Brom vorhanden ist. Ebenso ist es möglich, das Substrat II zusammen mit dem Bromierungsmittel und dem Initiator vorzulegen und die Reaktion mit der Zudosierung des Oxidationsmittels zu steuern.
Bei der Verwendung von Brom als Bromquelle wird im allgemeinen analog der beschriebenen Verfahrensweise vorgegangen, jedoch Wasser, Lösungsmittel und gegebenenfalls Initiator vorgelegt und Brom zudosiert. Das Substrat II kann bei dieser Fahrweise vorgelegt oder zudosiert werden.
Bei der Verwendung von stabilen Oxidationsmitteln können diese zusammen mit dem Substrat II vorgelegt werden und der Reaktionsablauf durch Zugabe der Bromkomponente gesteuert werden.

Die Bromierung läßt sich diskontinuierlich und bevorzugt kontinuierlich durchführen. Die kontinuierliche Fahrweise bietet den Vorteil der geringeren Dimensionierung der verwendeten Apparate und der damit verbundenen geringeren Vorratshaltung der das Substrat II enthaltenden Lösungen bei erhöhter Temperatur. Aufgrund der angesprochenen thermischen Instabilität von II bietet das kontinuierliche Verfahren damit einen sicherheitstechnischen Vorteil.

Nach Ende der Zudosierung wird das Reaktionsgemisch gewöhnlich noch 0,5 bis 3 Stunden bei der gewählten Reaktionstemperatur gehalten. Anschließend wird die organische Phase abgetrennt und ohne weitere Reinigung und Trocknung in der Alkylierungsstufe eingesetzt.

In der Alkylierungsstufe kommt das gleiche Lösungsmittel wie im Bromierungsschritt zum Einsatz. Möglich ist Zumischung eines polaren Lösungsmittels für diese Stufe.

Die 3-Hydroxypyrazole IV sind aus der Literatur bekannt oder können nach den dort beschriebenen Methoden hergestellt werden (z.B. Chem. Pharm. Bull. 19 (1971) 1389 -1394). Besonders vorteilhaft erhält man die Verbindungen IV nach den in der WO 97/03939, der EP-A 680 945 und der DE Anm. Nr. 19 652 516.0 beschriebenen Verfahren. Die gebildeten 3-Hydroxypyrazole können dabei z.T. als wäßrige Lösungen direkt in der anschließenden Alkylierung weiterverarbeitet werden.

Die Alkylierung von IV mit den o-Nitrobenzylbromiden III wird im allgemeinen bei Temperaturen von 20 bis 90°C und bevorzugt 40 bis 80°C durchgeführt.

Das o-Nitrobenzylbromid III wird im allgemeinen im Bezug auf IV in einem Molverhältnis von 0,9 bis 1,3, bevorzugt in einem Molverhältnis von 1,0 bis 1,2 eingesetzt.

Als Basen kommen allgemein anorganische Verbindungen wie Alkalimetall- und Erdalkalimetallhydroxide (z.B. Lithiumhydroxid, Natriumhydroxid, Kaliumhydroxid und Calziumhydroxid), Alkalimetall- und Erdalkalimetalloxide (z.B. Lithiumoxid, Natriumoxid, Calziumoxid und Magnesiumoxid), Alkalimetall- und Erdalkalimetallhydride (z.B. Lithiumhydrid, Natriumhydrid, Kaliumhydrid und Calziumhydrid), Alkalimetallamide (z.B. Lithiumamid, Natriumamid und Kaliumamid), Alkalimetall- und Erdalkalimetallcarbonate (z.B. Lithiumcarbonat und Calziumcarbonat) sowie Alkalimetallhydrogencarbonate (z.B. Natriumhydrogencarbonat), metallorganische Verbindungen, insbesondere Alkalimetallalkyle (z.B. Methyllithium, Butyllithium und Phenyllithium), Alkylmagnesiumhalogenide (z.B. Methylmagnesiumchlorid) sowie Alkalimetallund Erdalkalimetallalkoholate (z.B. Natriummethanolat, Natriumethanolat, Kaliumethanolat, Kalium-tert.-Butanolat und Dimethoxymagnesium), außerdem organische Basen, z.B. tertiäre Amine wie Trimethylamin, Triethylamin, Di-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Natriumhydroxid und Kaliumhydroxid.

Die Basen werden im allgemeinen äquimolar, im Überschuß oder gegebenenfalls als Lösungsmittel verwendet.

Es kann für die Umsetzung vorteilhaft sein, eine katalytische Menge eines Kronenethers (z.B. 18-Krone-6 oder 15-Krone-5) zuzusetzen.

Die Umsetzung kann auch in Zweiphasensystemen bestehend aus einer Lösung von Alkali- oder Erdalkalihydroxiden oder -carbonaten in Wasser und einer organischen Phase (z.B. aromatische und/oder halogenierte Kohlenwasserstoffe) durchgeführt werden. Als Phasentransferkatalysatoren kommen hierbei beispielsweise Ammoniumhalogenide und -tetrafluoroborate (z.B. Benzyltriethylammoniumchlorid, Benzyltributylammoniumbromid, Tetrabutylammoniumchlorid, Hexadecyltrimethylammoniumbromid oder Tetrabutylammoniumtetrafluoroborat) sowie Phosphoniumhalogenide (z.B. Tetrabutylphosphoniumchlorid und Tetraphenylphosphoniumbromid) in Betracht. Besonders bevorzugt sind Tetrabutylammoniumbromid, -hydroxid und -hydrogensulfat.

Es kann für die Umsetzung vorteilhaft sein, zunächst das 3-Hydroxypyrazol mit der Base in das entsprechende Hydroxylat umzusetzen, welches dann mit dem Benzylderivat umgesetzt wird.

Auch der Alkylierungsschritt läßt sich diskontinuierlich oder kontinuierlich durchführen.

### Verfahrensbeispiele

Am Beispiel der Synthese von 2-[(N-p-Chlorphenyl)3-pyrazolyloxymethyl]-nitrobenzol Ia durch
a) Bromierung von ortho-Nitrotoluol IIa und
b) Umsetzung des so gewonnenen ortho-Nitrobenzylbromids IIIa mit 3-Hydroxy-N-(p-Chlorphenyl)-pyrazol IVa
soll das erfindungsgemäße Verfahren näher erläutert werden.

### Beispiel 1

a) Herstellung von o-Nitrobenzylbromid
In einem 2,5 Liter Planschliffkolben mit Impellerrührer (300 Upm) und Stromstörer wurde eine Lösung von 6,6 g (1 mol.-% bezogen auf die eingesetzte Bromwasserstoffsäure) Azoisobutyronitril (AIBN) in 1350 g Chlorbenzol zusammen mit 620 g (3,6 mol) 47%iger Bromwasserstoffsäure vorgelegt. Man erwärmte den Reaktorinhalt auf 75°C. Nach Erreichen dieser Temperatur wurden die Zuläufe I und II mittels zweier Dosierpumpen zugefördert.
Zulauf I: eine Lösung von 26,2 g (4 mol.-%) AIBN in 548 g (4,0 mol) ortho-Nitrotoluol wurde kontinuierlich in 2 Stunden zugefahren; Zulauf II: 725 g (3,2 mol) 15%ige H₂O₂ wurde so zugefahren, daß kein überschüssiges Brom in der Lösung vorhanden war. Man benötigte hierfür ca. 2,5 Stunden.
Nach beendetem Zulauf ließ man 2 Stunden bei 75°C nachrühren, stellte dann den Rührer ab und trennte die Phasen bei 75°C. Man erhielt 2146,4 g organischer Phase mit folgender
Zusammensetzung (1t. quant. HPLC):

| | |
|---|---|
| 23,6 % | o-Nitrobenzylbromid |
| 8,4 % | o-Nitrotoluol |
| 7,1 % | o-Nitrobenzalbromid |

Ausbeute an o-Nitrobenzylbromid: 58,1 % bezogen auf eingesetztes o-Nitrotoluol
b) Herstellung von 2-[(N-p-Chlorphenyl)3-pyrazolyl-oxymethyl]-nitrobenzol
In einem 2,5 Liter Planschliffkolben mit Impellerrührer (420 Upm) und Stromstörer wurden 101,5 g (0,5 mol) 95,8%iges 3-Hydroxy-N-(p-Chlorphenyl)pyrazol, 875 g 5%ige wäßrige KOH und 40,25 g (0,025 mol) 20%ige wäßrige Tetrabutylammoniumbromid-Lösung vorgelegt. Zu dieser auf 80°C erwärmten, homogenen Mischung wurden 504 g der unter Stufe a) erhaltenen organischen Phase (entsprechend 0,55 mol o-Nitrobenzylbromid) innerhalb von 5 Minuten zudosiert. Eine Stunde wurde bei 80°C nachgerührt, anschließend wurde der Kesselinhalt bei verminderter Rührerdrehzahl von 200 UpM auf unter 10°C abgekühlt. Der Rückstand wurde filtriert, zweimal in Methanol aufgekocht und nochmals filtriert und schließlich im Vakuum bei 100 mbar getrocknet.
Ausbeute 141 g (85,6 %) des Wertprodukts mit einem Schmelzpunkt von 147°C.
Die folgenden Beispiele zeigen Verfahrensvarianten von Stufe a): Die jeweils erhaltene Lösung von o-Nitrobenzylbromid in Chlorbenzol kann wie in Beispiel 1 aufgezeigt direkt in den Alkylierungsschritt b) eingesetzt werden.

### Beispiel 2

### Verwendung von 2,2'-Azobis (2,4-dimethyl-valeronitril) als Initiator

Bei 45°C wurde eine Mischung aus 13,7 g (0,1 mol) o-Nitrotoluol, 25 g Chlorbenzol, 600 mg (2,7 mmol) V 65 (Fa. Wako; 2,2'-Azobis (2,4-dimethyl-valeronitril)), 300 mg H₂SO₄ und 16,4 g (0,15 mol) 30%igem Wasserstoffperoxid vorgelegt. Es wurden 10 g 47%ige Bromwasserstoffsäure in 75 min zugetropft und weitere 75 min bei 45°C nachgerührt. Man gab weitere 5 g Bromwasserstoffsäure zu und rührte 12 h bei Raumtemperatur. Dann gab man 3 g Bromwasserstoffsäure und in zwei Portionen 15,86 g einer Lösung aus Chlorbenzol und V 65 (insgesamt 15 g Chlorbenzol + 0,86 g (3,9 mmol) V 65) zu.
Das qualitative HPLC der organischen Phase zeigte nach Reaktionsende die folgende Zusammensetzung (Angabe in Flächenprozenten):

| | |
|---|---|
| 52,6 % | o-Nitrobenzylbromid |
| 35,5 % | o-Nitrotoluol |
| 4,3 % | o-Nitrobenzalbromid |
| 6,5 % | Chlorbenzol |

### Beispiel 3

Brom als Bromierungsmittel

Ein Gemisch aus 122,7 g Chlorbenzol, 45,5 g Wasser und 0,6 g (1 mol.-%) AIBN wurde vorgelegt und auf 75°C erhitzt. Nach Erreichen der Temperatur tropfte man eine Lösung von AIBN in 49,8 g (0,36 mol) o-Nitrotoluol innerhalb von 1 Stunde und parallel insgesamt 44,1 g (0,28 mol) Brom so zu, daß die Reaktionslösung permanent entfärbt (hellgelb-hellorange) blieb. Nach Ende der Zugabe wurde 1 Stunde bei 75°C gerührt. Man trennte die organische Phase bei 75°C ab.
174,5 g organische Phase der folgenden Zusammensetzung:

| | |
|---|---|
| 25,1 % | o-Nitrobenzylbromid |
| 11,3 % | o-Nitrotoluol |
| 3,1 % | o-Nitrobenzalbromid |

Ausbeute an o-Nitrobenzylbromid: 50,7 % bezogen auf eingesetztes o-Nitrotoluol.

### Beispiel 4

### Bromierung von o-Nitrotoluol im kontinuierlichen Prozeß

Zuläufe pro Stunde:

| Zulauf I: | | |
|---|---|---|
| 54,8 g | (0,4 mol) | o-Nitrotoluol |
| 3,3 g | (5 mol.-%) | AIBN (α,α'-Azoisobutyronitril) |
| 135 g | | Chlorbenzol |
| | | |

| Zulauf II: | | |
|---|---|---|
| 81,6 g | (0,36 mol) | 15%ige Wasserstoffperoxid-Lösung |
| | | |

| Zulauf III: | | |
|---|---|---|
| 62 g | (0,36 mol) | 47%ige Bromwasserstoffsäure |

In einer Rührkesselkaskade, bestehend aus 3 Rührkesseln (ca. 300 ml Füllvolumen), die im freiem Überlauf miteinander verbunden waren, wurden bei 75°C Innentemperatur und 300 UpM in den ersten Reaktor parallel die Zuläufe I bis III (getaucht) mittels waagengesteuerter Dosierpumpen gefahren. In einer nachgeschalteten Settlerstufe wurden bei 75°C die beiden Phasen ebenfalls kontinuierlich getrennt.

Beim Betrieb der Anlage über einen Zeitraum von 18 Stunden erhielt man:
3752,2 g organische Phase mit der folgenden Zusammensetzung:

| | |
|---|---|
| 21,5 % | o-Nitrobenzylbromid |
| 8,9 % | o-Nitrotoluol |
| 4,9 % | o-Nitrobenzalbromid |

### Ausbeute an o-Nitrobenzylbromid: 51.9 % bezogen auf eingesetztes o-Nitrotoluol

### Beispiel 5 verdünntere Fahrweise

Ein Gemisch aus 1500 g Chlorbenzol, 3,3 g (1 mol.-%) AIBN und 310,2 g (1,8 mol) 47%ige Bromwasserstoffsäure wurde vorgelegt und auf 75°C erhitzt. Nach Erreichen der Temperatur tropfte man eine Lösung von 13,1 g (4 mol.-%) AIBN in 274 g (2 mol) o-Nitrotoluol innerhalb von 2 Stunde und parallel insgesamt 408 g (1,8 mol) 15%iges Wasserstoffperoxid so zu, daß die Reaktionslösung permanent entfärbt (hellgelb-hellorange) blieb. Nach Zugabeende wurde 1 Stunde bei 75°C nachgerührt. Man trennte die organische Phase bei 75°C ab.

Es verbleiben:
1916,2 g organische Phase mit der folgenden Zusammensetzung:

| | |
|---|---|
| 14,2 % | o-Nitrobenzylbromid |
| 4,0 % | o-Nitrotoluol |
| 2,4 % | o-Nitrobenzalbromid |

### Ausbeute an o-Nitrobenzylbromid: 63 % bezogen auf eingesetztes o-Nitrotoluol

### Vergleichsbeispiele

### 1. Umsetzung von o-Nitrobenzalbromid mit 3-Hydroxy-N-(p-chlorphenyl)pyrazol

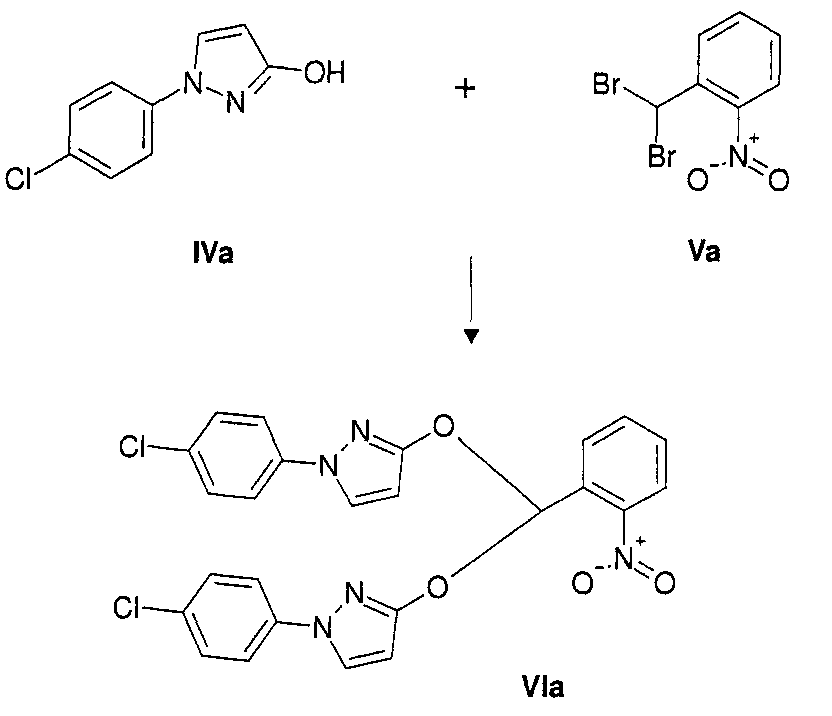

Eine Mischung von 6,8 g N-( p-Chlorphenyl)-3-hydroxypyrazol in 53,7 g (5%iger) Kalilauge wurde mit Tetra-n-butylammoniumbromid versetzt. Man gab eine Lösung von 5 g o-Nitro-benzalbromid in 20 g Chlorbenzol zu und erhitzte auf 80°C nach einer Nachrührzeit von 90 min kühlte man auf 5°C ab und saugte den ausgefallenen Feststoff ab. Dieser wurde mit kaltem MeOH gewaschen und anschließend im Vakuum bei 50°C getrocknet. Man erhielt so 6 g der Bis-pyrazolylverbindung VIa als bräunlichen Feststoff.

Aus der Mutterlauge wurden weitere 2 g Rückstand isoliert, der laut GC noch zu 80 % VIa enthielt.

### 2. Umsetzung eines Gemisch aus o-Nitrobenzalbromid und o-Nitrobenzylbromid mit 3- Hydroxy-N-(p-chlorphenyl)pyrazol

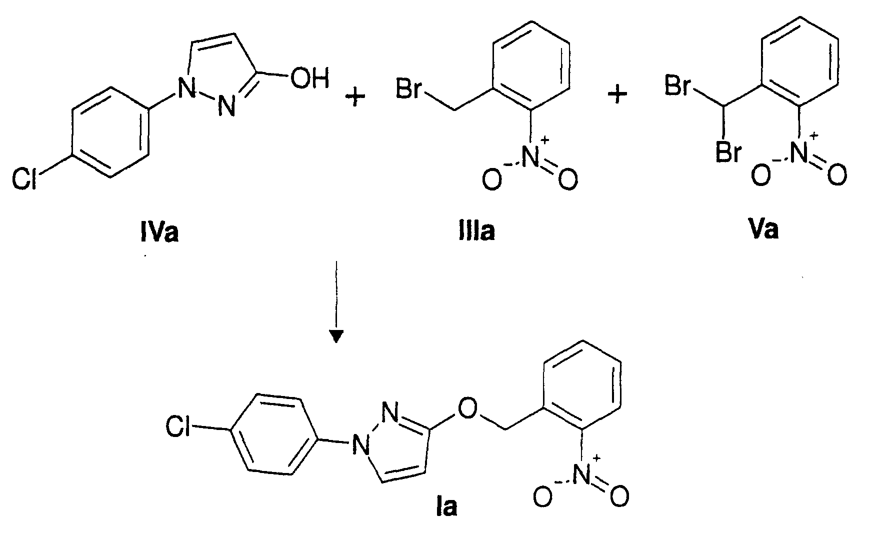

Eine Lösung von 9,9 g (51 mmol) 97,2%iges N-(p-Chlorphenyl)-3-hydroxypyrazol in 69,6 g 5%iger KOH wurde mit 0,8 g Tetra-nbutylammoniumbromid versetzt und auf 80°C erhitzt. Bei dieser Temperatur gab man eine Lösung von 10,7 g (49,5 mmol) o-Nitrobenzylbromid und 12,9 g (43,8 mmol) o-Nitrobenzalbromid zu und hielt 90 min bei dieser Temperatur. Das HPLC des Reaktionsgemisches zeigte, daß das o-Nitrobenzylbromid und Hydroxypyrazol zu dem gewünschten Benzylether Ia abreagiert hatten, während das o-Nitrobenzalbromid auch zum Reaktionsende noch unverändert vorlag.

Die isolierte Ausbeute an Ia nach Abkühlen, Absaugen und Nachwaschen mit Methanol lag in diesem Versuch bei 72,3 %.

Nach Vergleichsbeispiel 1 hätte man für o-Nitrobenzalbromid eine ähnliche Reaktivität wie für o-Nitrobenzylbromid erwartet. Überraschend jedoch verläuft die Alkylierung, wie in Vergleichsbeispiel 2 gezeigt, mit hoher Selektivität ab.

## Patentansprüche

1. Verfahren zur Herstellung von 2-(3-Pyrazolyl-oxymethylen)-nitrobenzolderivaten der Formel I 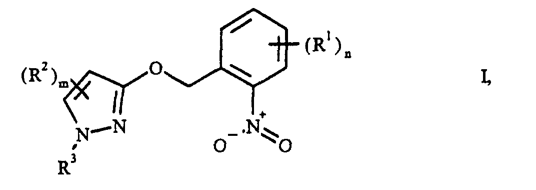 in der die Substituenten und Indices die folgende Bedeutung haben:
R¹ Halogen;
ggf. subst. Alkyl oder Alkoxy;
R² Cyano, Halogen, Alkyl, Halogenalkyl, Alkoxy, Alkylthio oder Alkoxycarbonyl;
R³ ggf. subst. Alkyl, Alkenyl oder Alkinyl;
ggf. subst. gesättigtes oder ein- oder zweifach ungesättigtes Carbocyclyl oder Heterocyclyl;
ggf. subst. Aryl oder Heteroaryl;
m 0, 1 oder 2, wobei die Substituenten R² verschieden sein können, wenn m größer als 1 ist;
n 0,1,2,3 oder 4, wobei die Substituenten R¹ verschieden sein können, wenn n größer als 1 ist;
durch Bromierung eines o-Nitrotoluols der Formel II 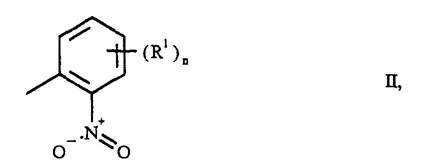 in der R¹ die vorgenannte Bedeutung besitzt, zum o-Nitrobenzylbromid der Formel III 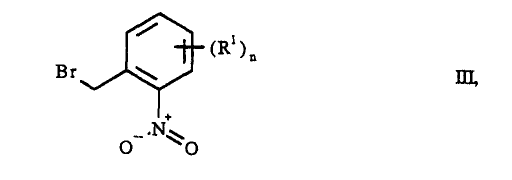 in Gegenwart eines unpolaren, aprotischen Lösungsmittels und anschließender Umsetzung der so erhaltenen Lösung von III mit einem 3-Hydroxypyrazol der Formel IV 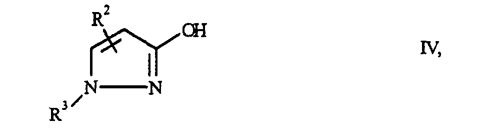 in der R² und R³ die vorgenannte Bedeutung haben, in Gegenwart einer Base, **dadurch gekennzeichnet, daß** die bei der Bromierung anfallende Lösung des o-Nitrobenzylbromids III im verwendeten Lösungsmittel direkt ohne Zwischenisolierung des o-Nitrobenzylbromids III mit IV weiter umgesetzt wird.

2. Verfahren gemäß Anspruch 1, **dadurch gekennzeichnet, daß** das o-Nitrobenzylbromid III in einem Molverhältnis von 1 bis 1,2 bezogen auf 3-Hydroxypyrazol IV, eingesetzt wird.

3. Verfahren gemäß Anspruch 1, **dadurch gekennzeichnet, daß** die Bromierung zum o-Nitrobenzylbromid III kontinuierlich durchgeführt wird.

4. Verfahren gemäß Anspruch 1, **dadurch gekennzeichnet, daß** sowohl die Bromierung zum o-Nitrobenzylbromid III als auch die anschließende Alkylierung zum 2-(3-Pyrazolyl-oxymethylen)-nitrobenzol I kontinuierlich durchgeführt werden.

5. Verfahren gemäß Anspruch 1, **dadurch gekennzeichnet, daß** die Bromierung zum o-Nitrobenzylbromid III in Gegenwart eines Azocarbonsäurenitrils oder Azocarbonsäureesters als Initiator durchgeführt wird.

6. Verfahren gemäß Anspruch 5, **dadurch gekennzeichnet, daß** die Bromierung zum o-Nitrobenzylbromid III mit Bromwasserstoffsäure, mit in Wasser gelösten anorganischen Bromiden oder mit elementarem Brom in Gegenwart eines Oxidationsmittels durchgeführt wird.

7. Verfahren gemäß Anspruch 6, **dadurch gekennzeichnet, daß** als Oxidationsmittel Wasserstoffperoxid eingesetzt wird.

8. Verfahren gemäß den Ansprüchen 1 bis 7, **dadurch gekennzeichnet, daß** bei der Bromierung und der Alkylierung in einem Zweiphasensystem, bestehend aus wäßriger und organischer Phase, gearbeitet wird.

## Claims

1. A process for preparing 2-(3-pyrazolyloxymethylene)nitrobenzene [sic] derivatives of the formula I 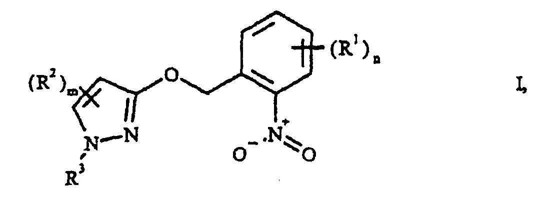 where:
R¹ is halogen;
unsubstituted or substituted alkyl or alkoxy;
R² is cyano, halogen, alkyl, haloalkyl, alkoxy, alkylthio or alkoxycarbonyl;
R³ is unsubstituted or substituted alkyl, alkenyl or alkynyl;
unsubstituted or substituted saturated or mono- or diunsaturated carbocyclyl or heterocyclyl;
unsubstituted or substituted aryl or hetaryl;
m is 0, 1 or 2, it being possible for the substituents R² to be different when m is greater than 1;
n is 0, 1, 2, 3 or 4, it being possible for the substituents R¹ to be different when n is greater than 1;
by bromination of an o-nitrotoluene of the formula II 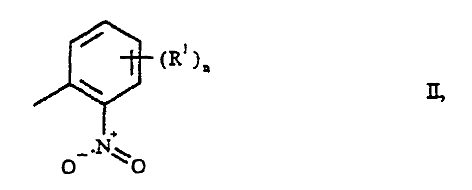 where R¹ has the abovementioned meaning, to give the o-nitrobenzyl bromide of the formula III 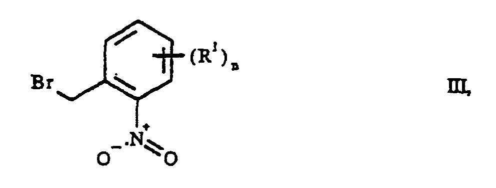 in the presence of a nonpolar, aprotic solvent and subsequent reaction of the resulting solution of III with a 3-hydroxypyrazole of the formula IV 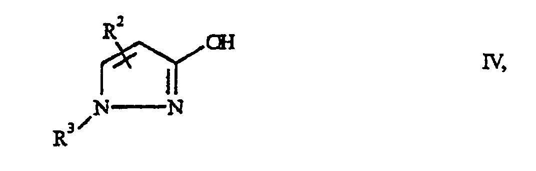 where R² and R³ have the abovementioned meanings, in the presence of a base, wherein the solution of the o-nitrobenzyl bromide III resulting from the bromination is reacted further with IV, in the solvent used, directly without intermediate isolation of the o-nitrobenzyl bromide III.

2. A process as claimed in claim 1, wherein the molar ratio of o-nitrobenzyl bromide III to 3-hydroxypyrazole IV employed is 1 - 1.2.

3. A process as claimed in claim 1, wherein the bromination to give the o-nitrobenzyl bromide III is carried out continuously.

4. A process as claimed in claim 1, wherein both the bromination to give o-nitrobenzyl bromide III and the subsequent alkylation to give the 2-(3-pyrazolyloxymethylene)nitrobenzene [sic] I are carried out continuously.

5. A process as claimed in claim 1, wherein the bromination to give the o-nitrobenzyl bromide III is carried out in the presence of an azo carbonitrile or azo carboxylic ester as initiator.

6. A process as claimed in claim 5, wherein the bromination to give the o-nitrobenzyl bromide III is carried out with hydrobromic acid, with inorganic bromides dissolved in water, or with elemental bromine in the presence of an oxidizing agent.

7. A process as claimed in claim 6, wherein hydrogen peroxide is employed as oxidizing agent.

8. A process as claimed in any of claims 1 to 7, wherein the bromination and the alkylation are carried out in a two-phase system consisting of aqueous and organic phase.

## Revendications

1. Procédé de préparation de dérivés du 2-(3-pyrazolyl-oxyméthylène)nitrobenzènes de formule I 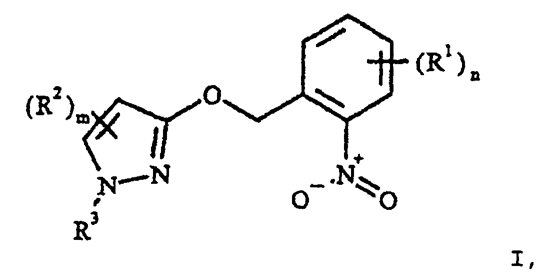 dans laquelle les substituants et les indices ont la signification suivante :
R¹ halogène ;
alkyle ou alcoxy, le cas échéant substitué ;
R2 cyano, halogène, alkyle, halogénoalkyle, alcoxy, alkylthio, ou alcoxycarbonyle ;
R3 alkyle, alcényle ou alcynyle, le cas échéant substitué ; carbocyclyle ou hétérocyclyle, le cas échéant, substitué, saturé ou une fois ou deux fois insaturé ;
aryle ou hétéroaryle, le cas échéant substitué ;
m 0, 1 ou 2, les substituants R² pouvant être différents, si m est supérieur à 1 ;
n 0, 1, 2, 3 ou 4, les substituants R¹ pouvant être différents, si n est supérieur à 1 ;
par bromation d'un o-nitrotoluène de formule II 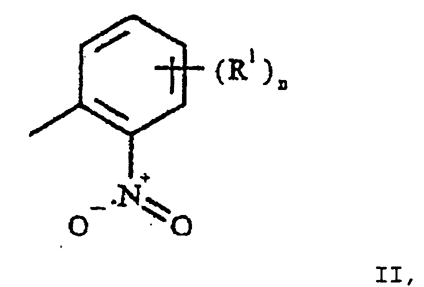 dans laquelle R¹ possède la signification précédente, pour former le bromure d'o-nitrobenzyle de formule III 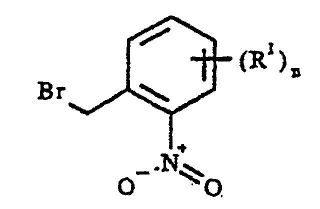 en,présence d'un solvant non polaire, aprotique et par réaction subséquente de la solution ainsi obtenue de III avec un 3-hydroxypyrazole de formule IV 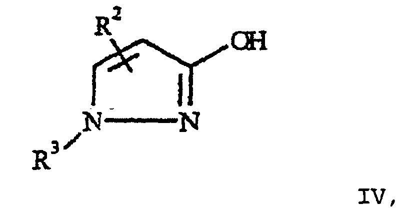 dans laquelle R² et R³ ont la signification précédente, en présence d'une base, **caractérisé en ce que** la solution du bromure d'o-nitrobenzyle III, se formant lors de la bromation, entre encore en réaction dans le solvant utilisé, directement, sans isolation intermédiaire du bromure d'o-nitrobenzyle III, avec le produit IV.

2. Procédé selon la revendication 1, **caractérisé en ce que** le bromure d'o-nitrobenzyle III est utilisé dans un rapport molaire de 1 à 1,2, par rapport au 3-hydroxypyrazole IV.

3. Procédé selon la revendication 1, **caractérisé en ce que** la bromation pour former le bromure d'o-nitrobenzyle III est effectuée d'une manière continue.

4. Procédé selon la revendication 1, **caractérisé en ce que** non seulement la bromation pour former le bromure d'o-nitrobenzyle III, mais aussi l'alkylation subséquente pour former le 2-(3-pyrazolyl-oxyméthylène)-nitrobenzène I est effectuée d'une manière continue.

5. Procédé selon la revendication 1, **caractérisé en ce que** la bromation pour former le bromure d'o-nitrobenzyle III est effectuée en présence d'un nitrile azocarboxylique ou d'un ester azocarboxylique en tant qu'amorceur.

6. Procédé selon la revendication 5, **caractérisé en ce que** la bromation pour former le bromure d'o-nitrobenzyle III est effectuée à l'aide d'acide bromhydrique, à l'aide de bromures inorganiques dissous dans l'eau ou à l'aide de brome élémentaire en présence d'un agent oxydant.

7. Procédé selon la revendication 6, **caractérisé en ce que** l'on utilise, en tant qu'agent oxydant, le peroxyde d'hydrogène.

8. Procédé selon les revendications 1 à 7, **caractérisé en ce que**, lors de la bromation et de l'alkylation, l'on travaille dans un système à deux phases, se composant d'une phase aqueuse et d'une phase organique.
